# EUROPÄISCHE PATENTSCHRIFT

(11) **EP 2 750 775 B1**
(45) Veröffentlichungstag und Bekanntmachung des Hinweises auf die Patenterteilung: **09.12.2020**
(21) Anmeldenummer: 12748706.4
(22) Anmeldetag: 23.08.2012
(51) Int. Cl.: B01D 3/00, C07C 319/20, C07C 323/58, C07D 233/76

(54) **VERFAHREN ZUR HERSTELLUNG EINES METHIONINSALZES**
METHOD FOR PRODUCING A METHIONINE SALT
PROCÉDÉ DE PRODUCTION D'UN SEL DE MÉTHIONINE

(30) Priorität: 30.08.2011 US 201161529013 P
(43) Veröffentlichungstag der Anmeldung: 09.07.2014
(73) Patentinhaber: Evonik Operations GmbH, 45128 Essen (DE)
(72) Erfinder: HASSELBACH, Hans, Joachim, 63571 Gelnhausen (DE); KÖRFER, Martin, 63796 Kahl (DE); GRÜNER, Christof, P, Mobile, AL 36609 (US); HANRATH, Franz, H, Mobile, AL 36695 (US); STOCK, Jürgen, 60599 Frankfurt (DE); GANGADWALA, Jignesh, Mobile, AL 36695 (US); KRULL, Horst, 63456 Hanau (DE)
(74) Vertreter: Evonik Patent Association
(86) Internationale Anmeldenummer: PCT/EP2012/066375
(87) Internationale Veröffentlichungsnummer: WO 2013/030068

(56) Entgegenhaltungen:
- EP-A1- 1 978 007
- EP-A2- 0 780 370
- US-B2- 7 655 072

## Beschreibung

Die Erfindung betrifft ein Verfahren zur Herstellung von einem Methionin-Salz, insbesondere die Herstellung von einem Methionin-Salz ausgehend von den Vorstufen 3-Methylmercaptopropionaldehyd (MMP) und Cyanwasserstoff (HCN) oder ausgehend von wenigstens einer aus diesen Rohstoffen herstellbaren Komponente wie Methylmercaptopropionaldehyd-Cyanhydrin (MMP-CN). Insbesondere betrifft die Erfindung die alkalische Hydrolyse von 5-(2-Methylmercapto)-hydantoin in einer Kolonne. Die Erfindung betrifft weiterhin ein für dieses Verfahren geeignetes Reaktionssystem, das eine Reaktivrektifikationskolonne umfasst sowie die Verwendung des Reaktionssystems.

Die industrielle Synthese von racemischem Methionin (Gemisch aus je 50 % L-Methionin und D-Methionin) geht von petrochemischen Rohstoffen aus, insbesondere Propen, Schwefel, Methan und Ammoniak. Nach gängigen Verfahren wird so über die Vorstufen Acrolein, Methylmercaptan und Blausäure die Vorstufe 3-Methylmercaptopropionaldehyd hergestellt. Anschließend wird dieser Aldehyd mit Ammoniak, Kohlendioxid und Blausäure in 5-(2-Methylmercapto)-hydantoin umgewandelt, dessen alkalische Hydrolyse zu einem Alkalisalz des Methionins führt. Durch Neutralisation mit einer Säure wie Kohlendioxid oder Schwefelsäure erhält man racemisches Methionin, wovon jährlich mehrere 100.000 Tonnen hergestellt werden.

Ein herkömmliches Verfahren basiert auf dem Einsatz von im Kreis geführten alkalischen Kaliumsalzen zur Hydrolyse des 5-(2-Methylmercapto)-hydantoins. Unerwünschte Nebenprodukte führen zu neutralen Kaliumsalzen, die dann der alkalischen Hydrolysereaktion nicht mehr zur Verfügung stehen. Diese müssen aus der kaliumhaltigen Kreislauflösung ausgeschleust werden. Die damit verbundenen Kaliumverluste müssen durch den Einsatz von KOH ausgeglichen werden. Eine weitere Nebenreaktion führt zur Bildung von 4-Methylmercapto-2-hydroxybutansäure und damit zu einem Ausbeuteverlust. 4-Methylmercapto-2-hydroxybutansäure findet sich zudem als neutrales Kaliumsalz im Sumpf der Reaktivdestillation wieder und stört damit den alkalischen Kalium-Kreislauf. Dieses Nebenprodukt ist daher nicht geeignet, die Hydrolyse von 5-(2-Methylmercapto)-hydantoin zu unterstützen und muss aus dem Kalium-Kreislauf ausgeschleust werden, was mit weiteren Verlusten an Rohstoffen verbunden ist.

Aus dem Stand der Technik ist bekannt, dass Methionylmethionin (auch Methionin-Dipeptid genannt) ein Nebenprodukt der Methioninherstellung durch Hydrolyse von Hydantoin ist (z. B. EP 2 133 329 A2, EP 0839 804 B1) und auf folgende Weise entsteht (Formel I).

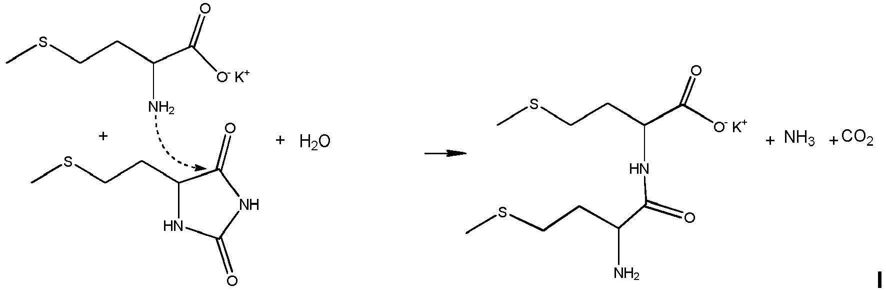

In EP 2 133 329 A2 wird zur Reduzierung der Methionin-Dipeptid Bildung eine zweistufige Hydantoin-Hydrolyse beschrieben. Dabei findet die erste Stufe in einem Strömungsrohr mit Gasauslass und die zweite Stufe in einem Rührreaktor statt, wobei Gesamtverweilzeiten von 20 - 60 Minuten angegeben sind. Man erhält so eine Produktverteilung von 91 mol-% Methionin und 9 mol-% Methionin-Dipeptid. Der hohe Anteil an Methionin-Dipeptid ist sehr unvorteilhaft und erfordert weitere Verfahrenschritte, um Ausbeuteverluste zu reduzieren. So wird in EP 2 186 797 A1 eine aufwendige Wärmebehandlung bei 150 -200°C der Konzentrate der Mutterlaugen aus der Methionin-Kristallisation beschrieben, um Methionin-Dipeptid hydrolytisch zu Methionin zurückzuspalten. Dazu muss die Mutterlauge nach der Kristallisation, die bei 20°C stattfindet, wieder auf die erforderlichen hohen Temperaturen erhitzt werden, was energetisch ungünstig ist.

In JP 2006-206534 A wird die Abreicherung von NH₃ aus einer in einem zusätzlichen Verfahrensschritt durch Hydrolyse von Hydantoin erzeugten Prozesslösung bei Normaldruck und daher bei etwa 100°C mittels einer Bodenkolonne beschrieben. Es handelt es sich hierbei um eine normale Destillation von NH₃, ohne Einfluss auf die Hydrolysereaktion. Nachteilig an diesem Vorgehen ist, dass das so entfernte NH₃ der Hydantoinsynthese nicht mehr zur Verfügung steht. Außerdem wird die so behandelte Lösung stark verdünnt, da Strippdampf mit einem Druck von 5 bar(ü) (entspr. 158°C) eingesetzt wird, was für die weitere Aufarbeitung nachteilig ist.

EP-A-1710232 und EP-A-1256571 beschreiben ein Verfahren zur Herstellung von D,L-Methionin aus 5-(2-Methylmercapto)-hydantoin, wobei die Verfahrensstufen 5-(2-Methylmercaptoethyl)-hydantoin-Bildung und Methioninat-Bildung (alkalische Hydrolyse) kontinuierlich betrieben und hintereinander verschaltet in einen gesamtkontinuierlich verlaufenden Prozess integriert werden können.

EP1978007 A1 offenbart einen Prozess, der die industrielle Herstellung von Diolen unter Verwendung einer Reaktivdestillationskolonne beschreibt.

EP780370 A2 offenbart ein Verfahren zur kontinuierlichen Herstellung eines Methionin-Salzes, wobei die Hydrolyse des Hydantoins zum Methionin-Salz in einer Reaktionsrektifikationskolonne erfolgt.

Im Stand der Technik ist bisher nicht beschrieben, wie eine Kolonne zur Hydrolyse von Met-Hydantoin technisch zu gestalten ist, um die Nebenproduktbildung zu minmieren. Weiterhin ist auch nicht beschrieben, wie der NH3-Kreislauf zwischen Hydantoin-Bildung und Hydantoin-Hydrolyse verlustfrei bei minimiertem Energieaufwand zu betreiben ist. Das Hydrolyse-Verfahren kann in einer mit Dampf beheizten Kolonne durchgeführt werden, wobei die 5-(2-Methylmercaptoethyl)-hydantoin-Lösung vorteilhaft am Kopf der Kolonne in der Geschwindigkeit kontinuierlich zugegeben wird, dass das Hydrolyseprodukt, Kaliummethioninatlösung, am Boden der Kolonne nach quantitativem Ablauf der Hydrolyse entsprechend abgezogen werden kann. Die Mutterlauge kann nach der Methionin-Feststoffabtrennung wieder eingesetzt werden. Die gasförmigen Bestandteile (Wasserdampf, Ammoniak und Kohlendioxid) können am Kopf der Kolonne ausgeschleust und zur Wiederherstellung der wässrigen Ammoniak/Kohlendioxid-Lösung zur Herstellung des 5-(2-Methylmercaptoethyl)-hydantoins eingesetzt werden. Als besonders vorteilhaft zur Vermeidung von Nebenprodukten wird beschrieben, die Hydrolyse von Anfang an in Gegenwart von Alkali und Kohlendioxid durchzuführen, d. h., dass insbesondere eine Mischung von Alkaliverbindungen vorliegt, insbesondere Alkalihydrogencarbonat, Alkalicarbonat, Alkalihydroxid. Zur Erzielung eines vollständigen Umsatzes des wertvollen Ausgangsmaterials MMP zum Hydantoin wird im beschriebenen Verfahren ein molares Verhältnis von 1,005 - 1,02 mol/mol HCN/MMP eingestellt. Durch die Hydrolyse unumgesetzten Cyanwasserstoffs kommt es in der Kolonne zu einer Produktion von NH₃ durch folgende Reaktion (Formel II):

HCN + 2H₂O → NH₃ + HCOOH II

Die entstehende Ameisensäure findet sich als nicht flüchtiges Kaliumformiat im Sumpf der Reaktionskolonne wieder, verdrängt also die benötigten alkalischen Carbonate und stört damit den alkalischen Kalium-Kreislauf.

Es war daher Aufgabe der Erfindung, ein gegenüber dem Stand der Technik verbessertes Verfahren bereitzustellen, um ausgehend von den Rohstoffen Methylmercaptopropionaldehyd und Cyanwasserstoff oder ausgehend von wenigstens einer aus diesen Rohstoffen herstellbaren Verbindung wie Methylmercaptopropionaldehyd-Cyanhydrin ein Methionin-Salz herzustellen.

Die Aufgabe wird durch das erfindungsgemäße Reaktionssystem, seine erfindungsgemäße Verwendung und das erfindungsgemäße Verfahren gelöst.

Das Reaktionssystem weist die folgenden Merkmale auf:
1. Reaktionssystem zur Herstellung eines Methionin-Salzes, umfassend eine Reaktivrektifikationskolonne mit einer Wehrhöhe
   im Bereich von 100 bis 1000 mm, vorzugsweise im Bereich von 150 bis 700 mm,
   wobei
   die Bodenabstände im Bereich von 500 bis 1000 mm liegen,
   das Verhältnis Kolonnendurchmesser zu Wehrlänge im Bereich von 1,1 bis 1,3 liegt,
   das Verhältnis Querschnittsfläche zur gasdurchströmten Fläche im Bereich von 1,5 bis 2 liegt und
   die Bodenanzahl im Bereich von 15 bis 25, vorzugsweise im Bereich von 18 bis 20 liegt.
2. Reaktionssystem nach 1, wobei die Reaktivrektifikationskolonne eine Siebbodenkolonne, Schlitzbodenkolonne, Ventilbodenkolonne oder Glockenbodenkolonne ist.
3. Reaktionssystem nach 2, wobei bei der Reaktivrektifikationskolonne das Verhältnis Summe der Fläche aller Löcher / gasdurchströmten Fläche im Bereich von 0,04 bis 0,08 liegt und
   der Durchmesser der Einzellöcher im Siebboden im Bereich von 5 bis 10 mm liegt.
4. Reaktionssystem nach einem der vorstehenden Ziffern, wobei die Wehrhöhen eine mittlere Verweilzeit des jeweiligen Gemischs von weniger als 0,5 min pro Boden gewährleisten.
5. Reaktionssystem, wobei das Reaktionssystem weiter wenigstens einen Reaktivabsorber und optional einen Nachreaktor zur Herstellung von 5-(2-Methylmercaptoethyl)-hydantoin umfasst.
6. Reaktionssystem nach 5, wobei der Reaktivabsorber ein Strahlwäschersystem ist.
7. Reaktionssystem nach einem der Ziffern 1 bis 6, wobei Zirkon in der Reaktivrektifikationskolonne und/oder in dem Reaktivabsorber und/oder in dem Nachreaktor als Werkstoff für produktberührte Teile verwendet wird.

Das Verfahren weist die folgenden Merkmale auf:
1. Verfahren zur kontinuierlichen Herstellung eines Methionin-Salzes, wobei folgende Schritte durchgeführt werden:
   - Umsetzung von 3-Methylmercaptopropionaldehyd und Cyanwasserstoff oder von einer daraus herstellbaren Komponente, wobei eine 5-(2-Methylmercaptoethyl)-hydantoin-haltige Lösung erhalten wird;
   - alkalische Hydrolyse des erhaltenen 5-(2-Methylmercaptoethyl)-hydantoins zu einem Methionin-Salz in einer Reaktivrektifikationskolonne, wobei auf dem obersten Boden der Reaktivrektifikationskolonne nur die 5-(2-Methylmercaptoethyl)-hydantoin-haltige Lösung eingespeist wird und eine alkalische Kreislauflösung auf einem darunter liegenden Boden, bevorzugt auf dem 2. Boden von oben eingespeist wird.
2. Verfahren nach 1, wobei die alkalische Kreislauflösung ein Alkalicarbonat, vorzugsweise Kaliumcarbonat enthält.
3. Verfahren nach 1 oder 2, wobei Wasser, Ammoniak und CO₂ über Kopf aus der Reaktivrektifikationskolonne entfernt werden und das entfernte NH₃ vollständig oder teilweise in die Synthese des 5-(2-Methylmercaptoethyl)-hydantoins einkondensiert wird.
4. Verfahren nach einem der Ziffern 1 bis 3, wobei die Konzentration des Ammoniaks im Sumpf der Rektifikationskolonne weniger als 120 ppm, bevorzugt weniger als 100 ppm und am meisten bevorzugt weniger als 80 ppm beträgt.
5. Verfahren nach einem der Ziffern 1 bis 4, wobei die Umsetzung zum 5-(2-Methylmercaptoethyl)-hydantoin in einem Reaktivabsorber und anschließend in einem Nachreaktor, vorzugsweise in einem als Strömungsrohr ausgelegten Nachreaktor durchgeführt wird.
6. Verfahren nach einem der Ziffern 1 bis 5, wobei die Temperatur des Reaktionsgemischs am Auslauf der Reaktivrektifikationskolonne im Bereich von 180°C bis 190°C liegt.
7. Verfahren nach einem der Ziffern 1 bis 6, wobei die Temperatur der Gasphase am Kopf der Reaktivrektifikationskolonne im Bereich von 160°C bis 170°C liegt.
8. Verfahren nach einem der vorstehenden Ziffern 1 bis 7,
   wobei die alkalische Hydrolyse bei einem Druck im Bereich von 8 bar(ü) bis 10 bar(ü) durchgeführt wird.
9. Verfahren nach einem der Ziffern 1 bis 8, wobei als Heiz- und Strippmedium in der Reaktivrektifikationskolonne Wasserdampf verwendet wird.
10. Verfahren nach einem der Ziffern 1 bis 9, wobei das Verfahren in einem Reaktionssystem nach einem der Ziffern 1 bis 8 durchgeführt wird.

Ein weiterer Gegenstand der Erfindung ist die Verwendung des Reaktionssystems zur Herstellung von Methionin.

Auf die in den Unteransprüchen wiedergegebenen bevorzugten Ausgestaltungen der Erfindung wird ausdrücklich Bezug genommen.

Gegenstand der Erfindung ist ein Verfahren zur kontinuierlichen Herstellung eines Methionin-Salzes, wobei 3-Methylmercaptopropionaldehyd und Cyanwasserstoff (HCN) oder eine daraus herstellbaren Komponente umgesetzt werden, wobei eine 5-(2-Methylmercaptoethyl)-hydantoin-haltige Lösung erhalten wird und wobei eine alkalische Hydrolyse des erhaltenen 5-(2-Methylmercaptoethyl)-hydantoins zu einem Methionin-Salz in einer Reaktivrektifikationskolonne, durchgeführt wird, wobei auf dem obersten Boden der Reaktivrektifikationskolonne nur die 5-(2-Methylmercaptoethyl)-hydantoin-haltige Lösung eingespeist wird und eine alkalische Kreislauflösung auf einem darunter liegenden Boden, bevorzugt auf dem 2. Boden von oben eingespeist wird. Bevorzugt enthält die alkalische Kreislauflösung ein Alkalicarbonat, vorzugsweise Kaliumcarbonat.

Die Umsetzung der Komponenten 3-Methylmercaptopropionaldehyd, Cyanwasserstoff sowie Ammoniak und Kohlendioxid oder solcher Komponenten, aus denen die vorgenannten Komponenten herstellbar sind, zum 5-(2-Methylmercaptoethyl)-hydantoin erfolgt gegebenenfalls in Gegenwart von Wasser. Um einen möglichst vollständigen Umsatz von Methylmercaptopropionaldehyd zu erzielen, werden die Ausgangsstoffe Cyanwasserstoff und Methylmercaptopropionaldehyd bevorzugt im molaren Verhältnis von 1,005 - 1,02 mol/mol HCN/MMP eingesetzt.

Bevorzugte Ausgangsstoffe zur Herstellung des 5-(2-Methylmercaptoethyl)-hydantoin (auch Hydantoin-Derivat oder kurz Hydantoin genannt) sind 3-Methylmercaptopropionaldehyd, Cyanwasserstoff, Ammoniak und Kohlendioxid. Nebenprodukte dieser Reaktion sind die Komponenten 5-(2-Methylmercaptoethyl)-hydantoinsäureamid, 5-(Methylmercaptoethyl)-hydantoinsäure, Methioninamid und in Spuren neben anderen Komponenten 3-Methylmercaptopropionaldehydcyanhydrin. Diese können in der alkalischen Hydrolyse ebenso wie das Hauptprodukt zu Methionin umgesetzt werden. Ausgenommen ist das 3-Methylmercaptopropionaldehydcyanhydrin, das bei der Hydrolyse zu 4-Methylmercapto-2-hydroxybutansäure wird. Die genaue Zusammensetzung des bei der Hydantoin-Reaktion entstehenden Produktgemischs kann mittels HPLC aufgeklärt werden.

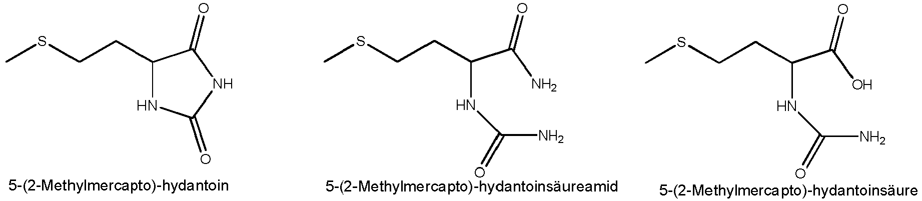

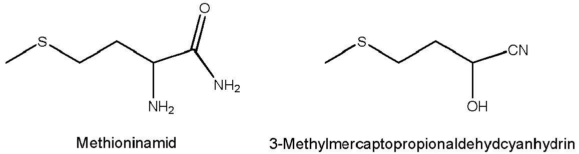

Vorteilhaft für einen vollständigen Umsatz von MMP zum Hydantoin-Derivat ist es, wenn im Reaktivabsorber MMP und NH₃ in einem molaren Verhältnis von etwa 1 zu 3 eingesetzt werden, wobei die Basisgleichung der Hydantoin-Synthese wie folgt aussieht (Formel III):

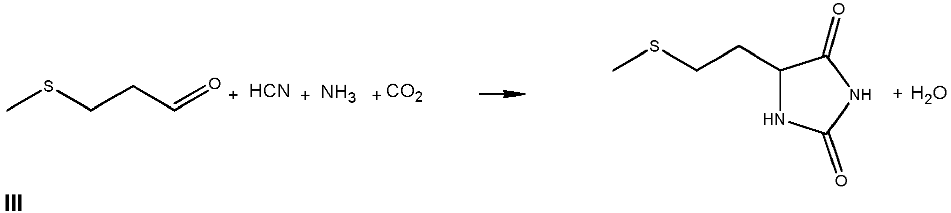

Wie aus Formel IV ersichtlich ist CO₂ als Reaktionspartner immer im Überschuss vorhanden, da bei Bildung des Methionin-Kalium-Salzes zusätzlich 0,5 Mole CO₂ aus Kaliumcarbonat freigesetzt werden.

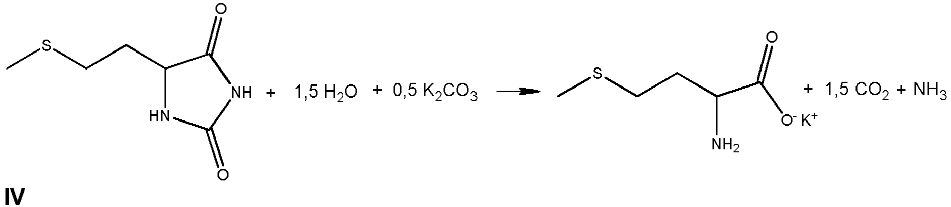

Weiterhin günstig für eine selektive Umsetzung von MMP mit NH₃, HCN und CO₂ in wässriger Phase zu Hydantoin ist es, wenn die Reaktionspartner im Moment des Aufeinandertreffens intensiv durchmischt werden. Daher wird als Reaktivabsorber bevorzugt ein Strahlwäschersystem eingesetzt. Die Menge an umgewälzter Prozesslösung beträgt dabei vorzugsweise das 18- bis 22-fache, bevorzugt das 20-fache der Summe der eingesetzten MMP- und HCN-Mengen. Damit sorgt der Strahlwäscher für einen sehr guten Stoffübergang von der Gasphase in die Flüssigphase.

Um einen vollständigen Umsatz von MMP zu Hydantoin in einer für die technische Anwendung akzeptablen Reaktionszeit zu erzielen, verlässt die Hydantoinreaktionsmischung den Strahlwäscher, indem sie über einen Nachreaktor, der als Strömungsrohr ausgelegt ist, geleitet wird, wobei die Verweilzeit im Strömungsrohr etwa 10 Minuten beträgt. Für eine möglichst hohe Hydantoin-Ausbeute ist es insbesondere wichtig die Bildung des Nebenproduktes 4-Methylmercapto-2-hydroxybutansäure (= Methionin-Hydroxyanaloges, MHA) zu vermeiden, die auftritt, wenn die Verweilzeit nicht ausreicht (Formel V):

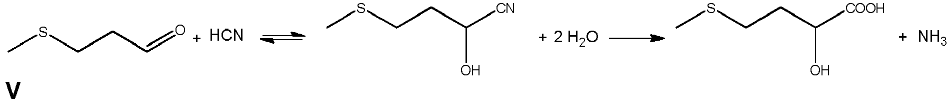

Durch die Nachreaktionsphase im Reaktionsrohr können die Verluste durch die Bildung von 4-Methylmercapto-2-hydroxybutansäure auf weniger als 1 mol% bezogen auf das eingesetzte MMP reduziert werden (Nachweis mittels HPLC).

Alternativ kann bei der Hydantoin-Herstellung auch zuvor synthetisiertes MMP-Cyanhydrin eingesetzt werden.

In einem nachfolgenden Schritt wird das Hydantoin-Derivat in der alkalischen Hydrolyse zu Methionin umgesetzt. Methionin bedeutet racemisches Methionin, das auch als D,L-Methionin bezeichnet wird. Bevorzugt wird dieser Schritt des erfindungsgemäßen Verfahren in einer Siebbodenkolonne durchgeführt, die als Reaktivdestillation betrieben wird.

Die in dem beanspruchten Verfahren betriebene Reaktionsdestillationskolonne, die vorzugsweise mit Siebböden ausgestattet ist, bewirkt neben der sehr effektiven Abdestillation des Ammoniaks, vor allem eine sehr vorteilhafte Reaktionsführung zur alkalischen Hydrolyse des Hydantoins unter Bildung des Kaliumsalzes des Methionins. Diese erfolgt gemäß der oben dargestellten Formel IV.

Die Erfindung bietet überdies den Vorteil, dass die zum Betrieb des gekoppelten Systems Reaktionsdestillation-Reaktionsabsorber benötigte NH₃-Menge nicht separat bereitgestellt und dosiert werden muss, sondern im System zirkuliert. Bevorzugt beträgt die Konzentration des Ammoniaks im Sumpfprodukt weniger als 120 ppm, weiter bevorzugt weniger als 100 ppm und am meisten bevorzugt weniger als 80 ppm. Durch diese Betriebsweise ist es vorteilhafterweise möglich im "steady state" völlig auf eine externe NH₃-Dosierung zu verzichten.

Es hat sich gezeigt, dass bei der Durchführung der Hydantoin-Hydrolyse in einer als Siebbodenkolonne ausgelegten Reaktionsdestillation mehrere vorteilhafte Effekte erzielt werden können.

Intensive eigene Untersuchungen der Erfinder zum Reaktionsmechanismus der Hydantoin-Hydrolyse haben überraschenderweise gezeigt, dass sich zunächst ein stabiles Zwischenprodukt bildet, nämlich das Kaliumsalz der Hydantoinsäure, gemäß folgender Gleichung (Formel VI).

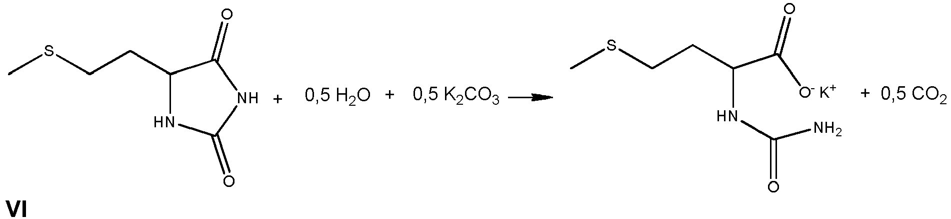

Der weitere hydrolytische Abbau der Hydantoinsäure erfolgt gemäß folgender Gleichung (Formel VII):

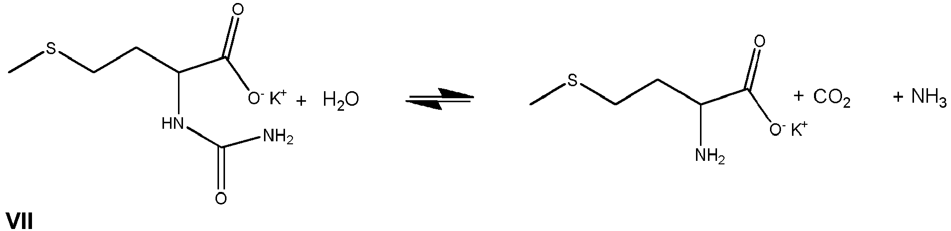

Da das gebildete Kalium-Salz über die Gleichgewichtsreaktion wieder zum Salz der Hydantoinsäure zurückreagieren kann, ist es für einen möglichst vollständigen Umsatz bei technisch erforderlichen kurzen Reaktionszeiten von großem Vorteil, wenn während der Reaktion effektiv Ammoniak und CO₂ aus der flüssigen Phase entfernt werden. Dazu wird in einer Ausführungsform eine Siebbodenkolonne verwendet. Zur Steuerung eines stabilen NH₃-holdups im beanspruchten Reaktionsdestillations-Reaktionsabsorber-System wird daher in einer weiteren Ausführungsform gezielt aus dem gasförmigen Kopfprodukt ein kleiner Teilstrom entnommen und entsorgt. Auf diese Weise gelingt es den entstehenden NH₃-Überschuss umweltfreundlich und unter Vermeidung des Verlustes wertvoller Rohstoffe aus dem System abzuführen.

Erfindungsgemäß werden bevorzugt über Kopf Wasser, Ammoniak (NH₃) und CO₂ abdestilliert und anschließend die gesamte NH₃-Menge oder ein Teil davon in die Hydantoin-Synthese einkondensiert, die bevorzugt als Reaktivabsorber ausgelegt ist, besonders bevorzugt als Reaktivabsorber, der ohne Verluste von NH₃ im Abgas betrieben wird. Im Strom aus dem Sumpf der Reaktivdestillation befindet sich das Alkalisalz, bevorzugt das Kaliumsalz des Methionins, das wie im Stand der Technik bekannt weiter zum Methionin aufgearbeitet wird.

Als Heiz- und Strippmedium wird bevorzugt Wasserdampf verwendet, der unter Druck unterhalb des untersten Siebbodens eingespeist wird. Menge, Geschwindigkeit und Temperatur des Dampfstroms werden vorzugsweise derart gesteuert, dass im Auslauf der Reaktionsdestillationskolonne eine Temperatur von 180°C - 190°C erreicht wird, während die Gasphase am Kopf die Kolonne mit einer Temperatur von 160°C - 170°C verlässt. Dem genannten Temperaturbereich entspricht ein Druckbereich von 8-10 bar(ü). Die Dampfmenge ist darüber hinaus abhängig von den gewünschten Durchsätzen.

Die Erfindung betrifft weiterhin ein Reaktionssystem zur Herstellung von Methioninsalz, das eine Reaktivrektifikationskolonne mit einer Wehrhöhe im Bereich von 100 bis 1000 mm (auch Reaktivdestillationskolonne oder Reaktivdestillation genannt) umfasst.

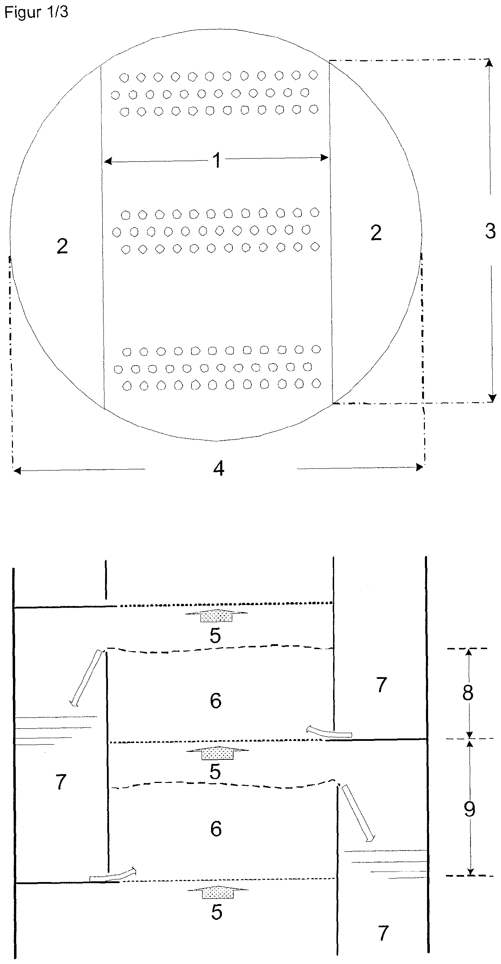

Die erfindungsgemäße Ausführungsform weist die folgende konstruktiven Merkmale auf (s. Figur 1):
Wehrhöhe **8:** 100 - 1000 mm, vorzugsweise 150 - 700 mm
Bodenabstände **9:** 500 - 1000 mm
Verhältnis: Kolonnendurchmesser **4**/Wehrlänge **3:** 1,1 - 1,3
Verhältnis: Querschnittsfläche / gasdurchströmten Fläche: 1,5 - 2
Bodenanzahl: 15 - 25, insbesondere 18 - 20.

Die Querschnittsfläche lässt sich mit Hilfe des Kolonnendurchmessers **4** errechnen. Die gasdurchströmte Fläche erhält man, wenn man von der Querschnittsfläche die Flächen der beiden Ablaufsegmente **2** subtrahiert.

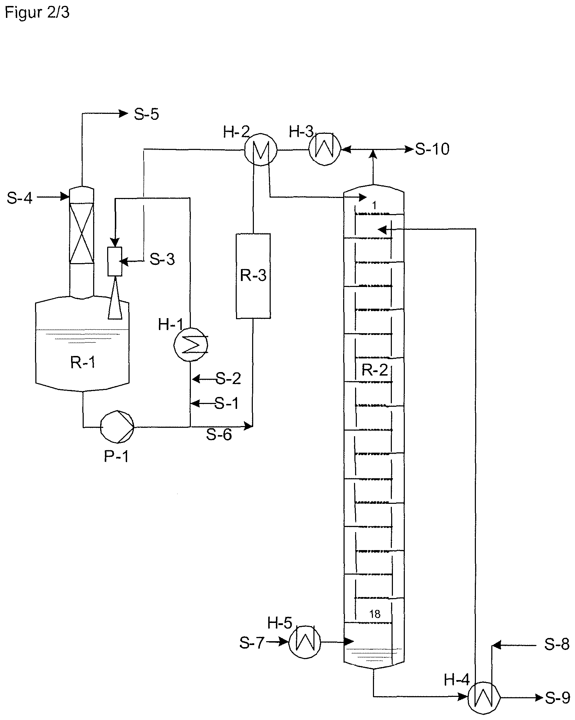

Handelt es sich bei der Kolonne um eine Siebbodenkolonne (s. Figur 1 und 2) sind folgende Merkmale bevorzugt:
Verhältnis: Summe der Fläche aller Löcher / gasdurchströmten Fläche: 0,04 - 0,08 Durchmesser der Einzellöcher im Siebboden 5-10 mm.

In der Figur 1 ist oben schematisch die Draufsicht eines Siebbodens und unten in einem Ausschnitt die Seitenansicht einer Kolonne einer bevorzugten Ausführungsform dargestellt. In der Seitenansicht erkennt man die Dampfphasen **5,** Flüssig-/Dampfphasen **6** und die Flüssigphasen **7** an den einzelnen Böden.

Bevorzugt können die Bödenabstände den unterschiedlichen Reaktionsphasen in der Kolonne angepasst werden. In einer Ausführungsform wird in der oberen Hälfte der Kolonne die Bodenabstände und die Wehrhöhen bevorzugt kleiner gehalten, um das Ausstrippen von NH₃ und CO₂ zu beschleunigen, während in der unteren Hälfte die Bodenabstände und Wehrhöhen größer sind, um durch mehr Verweilzeit den Umsatz zu komplettieren.

In einer bevorzugten Ausführungsform liegt die Wehrhöhe bei den oberen Böden einer Kolonne im Bereich von 100 bis 200 mm, bevorzugt bei 150 mm, bei einem Bodenabstand im Bereich von 800 bis 1000 mm, bevorzugt bei 1000 mm, und die Wehrhöhe bei den mittleren Böden im Bereich von 400 bis 600 mm, bevorzugt bei 500 mm, bei einem Bodenabstand im Bereich von 700 bis 900 mm, bevorzugt bei 800 mm, und die Wehrhöhe bei den unteren Böden im Bereich von 600 bis 800 mm, bevorzugt bei 700 mm, bei einem Bodenabstand im Bereich von 800 bis 1000 mm, bevorzugt bei 1000 mm. Der Bodenabstand wird jeweils von einem Boden zu dem darüber liegenden Boden gemessen. Bei Kolonnen mit 15 bis 17 Böden zählen bevorzugt die Böden 1 bis 4 zu den oberen, die Böden 5 bis 11 zu den mittleren und die tieferen Böden zu den unteren Böden. Bei Kolonnen mit 18 bis 21 Böden zählen bevorzugt die Böden 1 bis 5 zu den oberen, die Böden 6 bis 12 zu den mittleren und die tieferen Böden zu den unteren Böden. Bei Kolonnen mit 22 bis 25 Böden zählen bevorzugt die Böden 1 bis 7 zu den oberen, die Böden 8 bis 16 zu den mittleren und die tieferen Böden zu den unteren Böden.

Die bevorzugte Kombination aus Reaktivrektifikationskolonne und Reaktivabsorber erlaubt in einzigartiger Weise die Bereitstellung von Prozessbedingungen, die die Herstellung von Methionin in ganz besonderem Maße wirtschaftlich attraktiv werden lässt.

Die Reaktivrektifikationskolonne kann an einem oder mehreren oder allen Böden Probenahmestellen aufweisen. Bevorzugt weist sie an jedem vierte, jedem dritten, jedem zweiten bis vierten oder dritten, besonders bevorzugt an jedem zweiten Boden Probenahmestellen auf. Die genommenen Proben werden bevorzugt abgekühlt und mittels Hochdruckflüssigkeitschromatographie (HPLC) auf Hydantoin, Hydantoinsäure, Methionin, Methionyl-methionin untersucht. Der NH₃-Gehalt kann mittels einer ionenselektiven Elektrode potentiometrisch bestimmt werden.

Es zeigt sich vor allem, dass es möglich ist, die mit Siebböden (s. Figur 1) ausgestattete Reaktivdestillation einer bevorzugten Ausführungsform mit einer Dampfmenge von weniger als 0,25 t Dampf pro t Prozesslösungen, die am Kolonnenkopf aufgegeben werden, zu betreiben. Dies ist völlig unerwartet, da bei den oben beschriebenen hohen Drücken in der Reaktivdestillation und dem oben beschriebenen großen NH₃-Kreislauf aus dem Kopf der Reaktivdestillation von etwa 0,4 t NH₃ pro t eingesetztem MMP mit wesentlich größeren NH₃-Verlusten über den Sumpf der Kolonne zu rechnen war. Eine Untergrenze für die eingesetzte Dampfmenge ergibt sich dann, wenn die sogenannte Regengrenze der Siebböden erreicht ist. Darunter versteht man den Effekt, dass ab einem bestimmten reduzierten Gasstrom von unten zunehmend Flüssigkeit direkt durch die Löcher des Siebbodens abfließt (regnet) und nicht mehr den vorgesehenen Weg über die Ablaufsegmente **2** (Downcomer) nimmt. Dadurch würde die Verweilzeit für die Hydrolysereaktion verloren gehen und damit die gewünschte Funktion der Reaktivdestillation gestört. In detaillierten Untersuchungen an dem vorliegenden Reaktionssystem wurde ermittelt, dass die Regengrenze bei 50 % der oben genannten spezifischen Dampfmenge liegt. Bevorzugt sind Dampfmengen im Bereich von 0,13 t Dampf pro t Prozesslösung bis 0,4 t Dampf pro t Prozesslösung, am meisten bevorzugt im Bereich von 0,20 t Dampf pro t Prozesslösung bis 0,25 t Dampf pro t Prozesslösung.

In einer bevorzugten Ausführungsform wird Zirkon in der Reaktivdestillation und/oder in dem Reaktivabsorber und/oder in dem Nachreaktor als Werkstoff für die produktberührten Teile verwendet. Hierdurch können nachhaltig Korrosionsschäden an den produktberührten Teilen vermieden werden. Die hier beanspruchte Verfahrenskombination aus Reaktivdestillation und Reaktivabsorption erweist sich vor dem Hintergrund, dass Zirkon ein kostenintensiver Werkstoff ist, als besonders vorteilhaft, da durch die enge Verknüpfung der beiden Verfahrensschritte die Anzahl und die Länge der verbindenden Rohrleitungen und die Anzahl von Pufferbehältern minimiert ist. Damit ist das beanspruchte Verfahren insgesamt sehr nachhaltig, da es den korrosiv bedingten Austrag von umweltschädlichen Schwermetallen wie Chrom und Nickel vermeidet. Weiterhin kann durch den engen Verbund der Prozess-Schritte die beim Betrieb der Kolonne anfallende Abwärme in idealer Weise zum Aufheizen der Zulaufströme und zusätzlich zum Betrieb der Eindampfeinheit genutzt werden.

Das erfindungsgemäße Reaktionssystem gewährleistet eine ausreichende Verweilzeit der Reaktionspartner in der Kolonne. Gleichzeitig ist es wegen der hohen Kosten für den Werkstoff Zirkon vorteilhaft, dass die Anzahl der Böden klein gehalten werden kann. Bevorzugt sind die Böden Siebböden. Andere gängige Bodenkonstruktionen (z. B. Schlitz-, Ventil- oder Glockenböden) sind anwendbar, haben aber den Nachteil, dass deren Herstellung aus dem Werkstoff Zirkon sehr schwierig ist. Es wird daher in dieser Anmeldung auf Siebböden und Siebbodenkolonnen Bezug genommen. Die Erfindung ist jedoch nicht darauf beschränkt, sondern bezieht sich gleichermaßen auf Schlitz-, Ventil- oder Glockenböden bzw. Schlitz-, Ventil- oder Glockenbodenkolonnen, sofern nicht ausdrücklich etwas anderes genannt ist.

In einer Ausführungsform erlaubt die Reaktivdestillationskolonne eine quantitative Hydrolyse des Hydantoins bei Temperaturen zwischen 160°C und 180°C im Druckbereich 8 bar(ü) bis 10 bar(ü) in einer Verweilzeit von weniger als 10 Minuten, wobei die Kolonne eine mittlere Verweilzeit von weniger als 0,5 Minuten pro Boden gewährleistet. Einen wesentlichen Einfluss auf die Verweilzeit des einzelnen Siebbodens hat dabei die Wehrhöhe (s. Figur 1). Es hat sich überraschenderweise gezeigt, dass in dem beanspruchten Reaktionssystem wesentlich größere Wehrhöhen möglich sind, als die die im Stand der Technik (s. z. B. Mersmann, Thermische Verfahrenstechnik, S. 222, Springer Verlag, 1980) beschrieben sind. Dort werden Wehrhöhen bis maximal 60 mm genannt, während im beanspruchten Fall Wehrhöhen bis 1000 mm eingesetzt werden. Damit gelingt in einer bevorzugten Ausführungsform eine Minimierung des Einsatzes von Zirkon als Werkstoff, bei einer gleichzeitig auf weniger als 100 ppm reduzierten NH₃-Konzentration im Auslauf der Kolonne.

Zur alkalischen Hydrolyse des Hydantoins wird bevorzugt die Mutterlauge aus der Fällung des Methioninsalzes verwendet. Die Mutterlauge enthält die Kaliumsalze überwiegend als KHCO₃. Diese wird anschließend bevorzugt aufkonzentriert, um CO₂ und Wasser abzutrennen, was zu einer Lösung mit hohem Kaliumcarbonat-Anteil und damit erhöhter Basizität führt, die vorteilhaft für die Hydrolysereaktion ist.

Die zum Betrieb dieses Eindampfschrittes benötigte Energie kann in besonders geeigneter Weise aus der Abwärme der Kombination Reaktivdestillation-Reaktivabsorption bezogen werden. In einer bevorzugten Ausführungsform wird die zur Erzeugung der Dampfmengen, die zum Betrieb der Reaktivdestillation erforderlich sind, benötigte Wassermenge aus dem Kondensat des Eindampfschrittes bezogen. Damit kann der gesamte Methionin-Herstellprozess weitestgehend abwasserfrei betrieben werden, was aus ökologischen Gründen von großem Vorteil ist.

Gemäß dem erfindungsgemäßen Verfahrens werden zur alkalischen Hydrolyse die Reaktionslösungen derart in die Reaktivrektifikationskolonne eingebracht, dass auf dem obersten Boden nur die das 5-(2-Methylmercaptoethyl)-hydantoin enthaltene Lösung eingespeist wird und die alkalische Kreislauflösung auf einem darunter liegenden Boden, bevorzugt auf dem 2. Boden von oben eingespeist wird (s. Figur 2). Damit werden aus der Hydantoinlösung zunächst bevorzugt NH₃, CO₂ und HCN ausgestrippt und in die Hydantoinreaktion zurückgeführt. Dadurch wird vor allem der Verlust an HCN gemäß der Formel II minimiert und gleichzeitig die Bildung von Kaliumformiat vermieden. Kaliumformiat ist als neutrales Salz nicht geeignet die Hydrolyse von Hydantoin zu unterstützen und muss daher aus dem Kalium-Kreislauf ausgeschleust werden. Die damit verbundenen Kaliumverluste müssen durch Einsatz von KOH ausgeglichen werden. Die sequentielle Einspeisung von Hydantoinlösung und alkalischer Kreislauflösung am Kopf der Reaktivdestillation vermeidet daher Kosten für Rohstoffe und Entsorgung.

In einer bevorzugten Ausführungsform wird unter Verwendung einer Reaktivdestillation mit Siebböden auf vorteilhafter Weise die Bildung von Nebenprodukten wie Methionin-Dipeptid unterdrückt. Da für die Entstehung des Methionin-Dipeptids das gleichzeitige Vorliegen der Ausgangsverbindung Hydantoin und des Kaliumsalzes des Methionins notwendig ist, ist zur Vermeidung dieser Reaktion eine effiziente Trennung der Reaktionspartner vorteilhaft. Dies geschieht in besonders geeigneter Weise in einer mit Siebböden ausgerüsteten Reaktivdestillation, da in diesem System eine Rückvermischung weitestgehend unterdrückt wird. Die Erfindung ist jedoch nicht darauf beschränkt, sondern bezieht sich gleichermaßen auf Schlitz-, Ventil- oder Glockenböden bzw. Schlitz-, Ventil- oder Glockenbodenkolonnen, sofern nicht ausdrücklich etwas anderes genannt ist. Auf diese Weise wird in der hydrolysierten Reaktionsmischung ein Verhältnis von 98 mol-% Met und 2 mol-% Met-Dipeptid vorgefunden, wobei eine Verweilzeit von weniger als 10 Minuten benötigt wird.

Das bevorzugt beanspruchte Verfahren der alkalischen Hydantoin-Hydrolyse in einer Siebbodenkolonne minimiert die Bildung des Nebenproduktes Met-Dipeptid durch das Ausnutzen von zwei synergistischen Effekten. Zum einen wird durch die Bodenkonstruktion die Rückvermischung weitgehend verhindert und dadurch die Met-Dipeptid-Bildung gehemmt, zum anderen wird durch den intensiven Strippvorgang die Basizität in der Reaktionslösung erhöht, was durch nachfolgende Reaktionsgleichung dargestellt ist.

CO₃²⁻ + H₂O → 2 OH⁻ + CO₂, gas VIII

Ein erhöhter Basenanteil wiederum beschleunigt die Spaltung von entstandenem Met-Dipeptid. Der Mechanismus ist in Formel IX dargestellt:

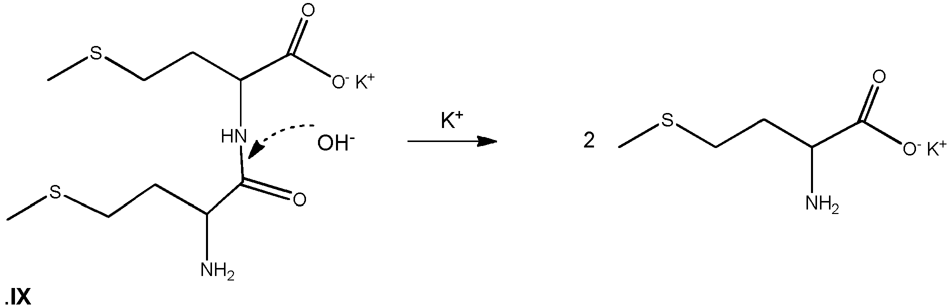

Damit wird verdeutlicht, dass die beanspruchte vielstufige Siebbodenkolonne sowohl die Met-Dipeptid-Bildung minimert als auch den hydrolytischen Abbau des Dipeptids unterstützt, was insgesamt Verluste, die mit der Nebenproduktbildung als auch deren Entsorgung verbunden sind, stark reduziert.

Wesentlich für einen effizienten Umsatz des Hydantoins zum Methionin in der beanspruchten Kolonne ist das Vorhandensein einer ausreichenden Menge an nicht flüchtigen basischen Verbindungen in der Reaktionsmatrix. Diese sind Kaliumsalze, wie KOH, Kaliumcarbonat, Kaliumhydrogencarbonat, Kalium-Methioninat, Kaliumsalz des Met-Dipeptids. Da diese Kaliumsalze sehr weitgehend rezykliert werden, zugleich aber in Nebenreaktionen auch Kaliumsalze starker Säuren entstehen (Formel II und Formel V) ist es wichtig den Basizitätsgrad im Kalium-Kreislauf zu überwachen und stabil zu halten.

Im beanspruchten kontinuierlich betriebenen Verfahren ist es daher wichtig den Basizitätsgrad insbesondere am Ausgang der Kolonne zu erfassen. Dazu wird eine Probe entnommen und diese nach Abkühlen auf Umgebungstemperatur einer klassischen Säure-Base-Titration mit einer Säure als Titrationsmittel unterzogen, wobei der Endpunkt der Titration zwischen pH 4 und 5 gefunden wird. Bevorzugt liegen die Basizitätswerte in der Reaktionsmatrix am Ende der Reaktion im Bereich von 2,2 - 2,8 mol Base pro kg verseifte Lösung, vorzugsweise bei 2,5 mol Base/kg verseifte Lösung. Niedrigere Basizitäten führen zu verstärkter Met-Dipeptidbildung und höheren NH₃-Konzentrationen im verseiften Produkt. Höhere Basizitätswerte bedeuten, dass bezogen auf die produzierte Met-Menge, ein größerer Kreislauf an alkalischen Kaliumsalzen im Verfahren betrieben werden muss. Dies ist energetisch zunehmend aufwendig und daher kontraproduktiv.

### BEISPIELE

### Beispiel 1

Zur weiteren Erläuterung der Erfindung dient Figur 2.

Im Reaktionssystem **R-1** findet die Herstellung von Hydantoin statt. Die Rohstoffe HCN und MMP werden über die Einspeisestellen **S-1** und **S-2** in die etwa 20-fachen Menge an Hydantoin-Reaktionslösung eingemischt, die durch die Pumpe **P-1** umgewälzt wird. Wärmetauscher **H-1** dient der Wärmeabfuhr und hält den Reaktorinhalt **R-1** bevorzugt bei einer Temperatur von 100°C - 120°C. Alternativ kann auch eine Vormischung aus MMP und HCN oder separat hergestelltes MMP-Cyanhydrin in den von Pumpe **P-1** umgewälzten Strom eingespeist werden. Der so erzeugte vereinigte Strom dient dann als Treibstrahl in einem Strahlmischer, der die teilkondensierten Leichtflüchter **(S-3)** aus dem Kopf der Reaktivdestillation **R-2** intensiv mit der Hydantoin-Prozesslösung in Kontakt bringt und in den Reaktor **R-1** zurückführt. Über den Strom **S-5,** der mit Wasser **(S-4)** NH₃-freigewaschen wird, verlässt ein CO₂-haltiger Strom den Reaktor **R-1.** Dieser CO₂-Strom fällt an, weil gemäß Formel IV bei der Hydantoin-Hydrolyse in Gegenwart von Kaliumcarbonat stöchiometrisch 0,5 mol CO₂ pro Mol gebildetem Methionin mehr entstehen als für die Hydantoin-Bildung gemäß Formel III benötigt werden. Der so gewonnene NH₃-freie CO₂-Strom wird sinnvoller Weise wie in US 7,655,072 B2 beschrieben wieder in den Aufarbeitungsteil zur Isolierung von kristallinem Methionin zurückgeführt, da dort zur Neutralisation der alkalischen Prozesslösung CO₂ eingesetzt wird.

Der flüssige hydantoinhaltige Strom **S-6** wird über einen Nachreaktor **R-3** geführt, um den Umsatz zu vervollständigen. Gleichzeitig wird damit der Eintrag von MMP-Cyanhydrin in den Hydrolysereaktor **R-2** verhindert, so dass dort die störende Bildung von 4-Methylmercapto-2-hydroxybutansäure verhindert wird (siehe Formel V). Um den Energiebedarf in der Reaktionsdestillation **R-2** zu minimieren, wird der hydantoinhaltige Prozessstrom **S-6** vor dem Eintritt auf den obersten Boden der Siebboden-Kolonne **R-2** mittels Wärmetauscher **H-2** bevorzugt auf 130°C vorgewärmt. Auf Boden 1 der Reaktionsdestillation wird zunächst bevorzugt noch vorhandene HCN ausgetrieben. Das vermindert die sonst in der Reaktionsdestillation im alkalischen stattfindenden Hydrolyse der HCN zum Kaliumsalz der Ameisensäure (Formel II).

Die Hydrolyse des Hydantoins zu Methionin beginnt mit dem Zusammentreffen der hydantoinhaltigen Prozesslösung und dem Strom **S-8,** der aus den aufkonzentrierten Filtraten der Aufarbeitung zum Methioninsalz-Feststoff **S-9** stammt. Der Strom **S-8** übernimmt soviel Wärme wie möglich aus dem Sumpfprodukt der Reaktivdestillation (Wärmetauscher **H-4)** und wird so bevorzugt auf 170°C erhitzt. Mit Hilfe der Verdampfereinheit **H-5** wird in bevorzugter Weise der Dampf zum Betrieb der Reaktivdestillation erzeugt, indem als Wasserquelle Kondensate aus der Aufarbeitung (Strom **S-7)** eingesetzt werden.

Über Strom **S-10** wird vor allem der Ammoniak-Haushalt im beanspruchten gekoppelten System reguliert, da durch den Einsatz von überschüssiger HCN in der Hydantoin-Synthese und deren Hydrolyse zu NH₃ und Ameisensäure zusätzliche NH₃-Mengen entstehen, die an dieser Stelle sehr selektiv, d. h. ohne Verluste an Hydantoin oder Methionin, ausgeschleust werden können. Verdampfer **H-3** erzeugt aus Kesselspeisewasser Heizdampf der Druckstufe 3 bar(ü) (130°C), der bevorzugt in der Eindampfung der Methionin-Mutterlauge eingesetzt wird. Damit kann in idealer Weise Abwärme aus dem bei Temperaturen zwischen 170°C und 190°C betriebenen Reaktivdestillation in der weiteren Aufarbeitung genutzt werden, wodurch der gesamte Herstellungsprozess für Methionin nach dem beanspruchten Verfahren energetisch äußert günstig und daher wirtschaftlich attraktiv gestaltet ist.

### Beispiel 2

Eine Siebbodenkolonne mit einem Durchmesser von 1 Meter und 18 Siebböden wie in Figur 2 dargestellt wird zur kontinuierlichen Produktion von Methionin eingesetzt. Die Anordnung der Siebböden, ihre Abstände und Wehrhöhen ergibt sich aus folgender Tabelle:

| Boden-Nr. | Wehrhöhe [mm] | Bodenabstand [mm] |
|---|---|---|
| 1 bis 5 | 150 | 1000 |
| 6 bis 12 | 500 | 800 |
| 13 bis 18 | 700 | 1000 |

Der Behälter **R-1** wird mit einem hold up von 3 m³ betrieben, der Nachreaktor hat einen hold up von 1 m³. Über die Pumpe **P-1** wird eine Umwälzmenge von 42 t/h eingestellt. In diesen Strom werden bei **S-1** 442 kg/h HCN (16,92 kmol/h) und bei **S-2 1688** kg/h MMP (16,24 kmol/h) eingespeist. Über **S-3** wird ein Strom von 6063 kg/h mit einem NH₃-Gehalt von 11,6 Gew-% an Kondensaten aus dem Kopf der Reaktivdestillation in die umgewälzte Prozesslösung eingemischt. Überschüssiges CO₂-Gas (400 kg/h) verlässt Behälter **R-1** über eine Waschkolonne, die am Kopf **(S-4)** mit 770 kg/h Wasser beaufschlagt wird, wodurch der CO₂-Strom NH₃-frei gewaschen und dann rezykliert werden kann.

Mittels Kühler **H-1** wird die Temperatur im Ausgang von **R-1** bei 105°C einreguliert. Die Hydantoin-Reaktionslösung **(S-6)** wird zur Vervollständigung der Reaktion über den Nachreaktor **R-3** geführt, im Wärmetauscher **H-2** auf 130°C erhitzt und dann auf den obersten Boden der Siebbodenkolonne gespeist. Auf Boden 2 werden 14,14 t/h mit Wärmetauscher **H-4** auf 170°C erhitzte kaliumcarbonathaltige Prozesslösung **(S-8)** gepumpt, die folgende Komponenten enthält: 66 g/kg Met, 158 g/kg Kalium, 48 g/kg Met-Met, 6,5 g/kg MHA, 12,5 g/kg Formiat, 3,6 mol Base /kg an Basizität.

Der zum Betrieb erforderliche Dampfstrom (5470 kg/h) wird mittels Verdampfer **H-5 (S-7)** erzeugt und unterhalb des untersten Siebbodens eingespeist. Aus dem über Kopf destillierten Strom wird ein Teilstrom **S-10** (54 kg/h) abgezweigt und entsorgt.

Der Druck am Kopf der Siebbodenkolonne beträgt 8,2 bar(ü) und die Temperatur auf der Eingangsseite des Wärmetauschers **H-3** 165°C. Der Differenzdruck über alle Siebböden liegt bei 450 mbar.

Die Temperatur im Sumpf der Kolonne beträgt 189°C.
Am Ausgang der Kolonne fällt ein Strom **S-9** von 22,15 t/h an, der folgende Komponenten enthält.149 g/kg Met, 32 g/kg Met-Met, 101 g/kg Kalium, 8,2 g/kg.Formiat, 4,2 g/kg MHA und hat einen Basizitätswert von 2,5 mol Base/kg.

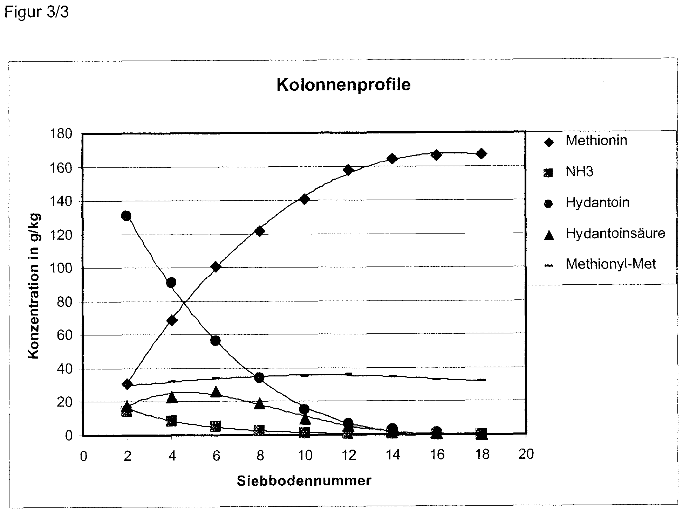

An den Böden 2, 4, 6, 8, 10, 12, 14, 16 und 18 ist die Siebbodenkolonne mit Probenahmestellen ausgerüstet. Die Proben werden sofort abgekühlt und mittels Hochdruckflüssigkeitschromatographie (HPLC) auf Hydantoin, Hydantoinsäure, Methionin, Methionyl-methionin untersucht. Der NH₃-Gehalt wird mittels einer ionenselektiven Elektrode potentiometrisch bestimmt. Die Zusammensetzung auf den verschiedenen Siebböden ist in folgender Tabelle und in Figur 3 zusammengefasst.

| | Methionin | NH₃ | \| Hydantoin | Hydantoinsäure | Methionyl-Met |
|---|---|---|---|---|---|
| Siebboden-Nr. | [g/kg] | [g/kg] | [g/kg] | [g/kg] | [g/kg] |
| 2 | 31 | 14,2 | 131 | 18 | 30 |
| 4 | 69 | 8,4 | 91 | 23 | 32 |
| 6 | 101 | 5 | 56 | 26 | 34 |
| 8 | 122 | 2,4 | 34 | 19 | 35 |
| 10 | 141 | 1,5 | 15,1 | 10 | 35,5 |
| 12 | 158 | 0,7 | 6,4 | 5 | 36 |
| 14 | 164 | 0,6 | 3,2 | 2,4 | 35 |
| 16 | 166 | 0,2 | 1,2 | 0,6 | 33 |
| 18 | 167 | 0,1 | 0 | 0 | 32 |

Zur Bestimmung der Verweilzeit wird der Flüssigkeitsinhalt im kontinuierlichen Betrieb bestimmt, indem zunächst die Ströme **S-6, S-7, S-8** und **S-9** gleichzeitig gestoppt werden. Es wird abgewartet bis kein Niveauanstieg im Kolonnensumpf mehr registriert wird und dann durch Abpumpen das Ausgangsniveau im Kolonnensumpf wieder hergestellt. Die Menge an abgepumpter Lösung beträgt 2,4 t. Da im kontinuierlichen Betrieb der Durchsatz bei 22,15 t/h liegt ergibt sich eine Verweilzeit der flüssigen Phase von 6,5 min.

## Patentansprüche

1. Reaktionssystem zur Herstellung eines Methionin-Salzes, umfassend eine Reaktivrektifikationskolonne mit einer Wehrhöhe
im Bereich von 100 bis 1000 mm, wobei
die Bodenabstände im Bereich von 500 bis 1000 mm liegen,
das Verhältnis Kolonnendurchmesser zu Wehrlänge im Bereich von 1,1 bis 1,3 liegt,
das Verhältnis Querschnittsfläche zur gasdurchströmten Fläche im Bereich von 1,5 bis 2 liegt und
die Bodenanzahl im Bereich von 15 bis 25, vorzugsweise im Bereich von 18 bis 20 liegt.

2. Reaktionssystem nach Anspruch 1, wobei die Wehrhöhe im Bereich von 150 bis 700 mm liegt.

3. Reaktionssystem nach Anspruch 1 oder 2, wobei die Reaktivrektifikationskolonne eine Siebbodenkolonne, Schlitzbodenkolonne, Ventilbodenkolonne oder Glockenbodenkolonne ist.

4. Reaktionssystem nach Anspruch 3, wobei bei der Reaktivrektifikationskolonne
das Verhältnis Summe der Fläche aller Löcher / gasdurchströmten Fläche im Bereich von 0,04 bis 0,08 liegt und
der Durchmesser der Einzellöcher im Siebboden im Bereich von 5 bis 10 mm liegt.

5. Reaktionssystem nach einem der vorstehenden Ansprüche, wobei die Wehrhöhen eine mittlere Verweilzeit des jeweiligen Gemischs von weniger als 0,5 min pro Boden gewährleisten.

6. Reaktionssystem, wobei das Reaktionssystem weiter wenigstens einen Reaktivabsorber und optional einen Nachreaktor zur Herstellung von 5-(2-Methylmercaptoethyl)-hydantoin umfasst.

7. Reaktionssystem nach Anspruch 5, wobei der Reaktivabsorber ein Strahlwäschersystem ist.

8. Reaktionssystem nach einem der Ansprüche 1 bis 7, wobei Zirkon in der Reaktivrektifikationskolonne und/oder in dem Reaktivabsorber und/oder in dem Nachreaktor als Werkstoff für produktberührte Teile verwendet wird.

9. Verfahren zur kontinuierlichen Herstellung eines Methionin-Salzes, wobei folgende Schritte durchgeführt werden:
- Umsetzung von 3-Methylmercaptopropionaldehyd und Cyanwasserstoff oder von einer daraus herstellbaren Komponente, wobei eine 5-(2-Methylmercaptoethyl)-hydantoin-haltige Lösung erhalten wird;
- alkalische Hydrolyse des erhaltenen 5-(2-Methylmercaptoethyl)-hydantoins zu einem Methionin-Salz in einer Reaktivrektifikationskolonne gemäß einem der Ansprüche 1-8, wobei auf dem obersten Boden der Reaktivrektifikationskolonne nur die 5-(2-Methylmercaptoethyl)-hydantoinhaltige Lösung eingespeist wird und eine alkalische Kreislauflösung auf einem darunter liegenden Boden, bevorzugt auf dem 2. Boden von oben eingespeist wird.

10. Verfahren nach Anspruch 9, wobei die alkalische Kreislauflösung ein Alkalicarbonat, vorzugsweise Kaliumcarbonat enthält.

11. Verfahren nach Anspruch 9 oder 10, wobei Wasser, Ammoniak und CO₂ über Kopf aus der Reaktivrektifikationskolonne entfernt werden und das entfernte NH₃ vollständig oder teilweise in die Synthese des 5-(2-Methylmercaptoethyl)-hydantoins einkondensiert wird.

12. Verfahren nach einem der Ansprüche 9 bis 11, wobei die Konzentration des Ammoniaks im Sumpf der Rektifikationskolonne weniger als 120 ppm, bevorzugt weniger als 100 ppm und am meisten bevorzugt weniger als 80 ppm beträgt.

13. Verfahren nach einem der Ansprüche 9 bis 12, wobei die Umsetzung zum 5-(2-Methylmercaptoethyl)-hydantoin in einem Reaktivabsorber und anschließend in einem Nachreaktor, vorzugsweise in einem als Strömungsrohr ausgelegten Nachreaktor durchgeführt wird.

14. Verfahren nach einem der Ansprüche 9 bis 13, wobei die Temperatur des Reaktionsgemischs am Auslauf der Reaktivrektifikationskolonne im Bereich von 180°C bis 190°C liegt.

15. Verfahren nach einem der Ansprüche 9 bis 14, wobei die Temperatur der Gasphase am Kopf der Reaktivrektifikationskolonne im Bereich von 160°C bis 170°C liegt.

16. Verfahren nach einem der vorstehenden Ansprüche 9 bis 15,
wobei die alkalische Hydrolyse bei einem Druck im Bereich von 8 bar(ü) bis 10 bar(ü) durchgeführt wird.

17. Verfahren nach einem der Ansprüche 9 bis 16, wobei als Heiz- und Strippmedium in der Reaktivrektifikationskolonne Wasserdampf verwendet wird.

18. Verfahren nach einem der Ansprüche 9 bis 17, wobei das Verfahren in einem Reaktionssystem nach einem der Ansprüche 1 bis 8 durchgeführt wird.

19. Verwendung eines Reaktionssystems nach einem der Ansprüche 1 bis 8 zur Herstellung von Methionin.

## Claims

1. Reaction system for production of a methionine salt, comprising a reactive-rectification column with a weir height in the range from 100 to 1000 mm, wherein the plate spacing is in the range from 500 to 1000 mm, the ratio of column diameter to weir length is in the range from 1.1 to 1.3, the ratio of the cross-sectional area to the area through which gas flows is in the range from 1.5 to 2 and the number of plates is in the range from 15 to 25, preferably in the range from 18 to 20.

2. Reaction system according to Claim 1, wherein the weir height is in the range from 150 to 700 mm.

3. Reaction system according to Claim 1 or 2, wherein the reactive-rectification column is a sieve plate column, perforated plate column, valve plate column or bubble plate column.

4. Reaction system according to Claim 3, wherein in the reactive-rectification column the ratio of total area of all holes / area through which gas flows is in the range from 0.04 to 0.08 and the diameter of the individual holes in the sieve plate is in the range from 5 to 10 mm.

5. Reaction system according to one of the preceding claims, wherein the weir heights ensure an average residence time of the respective mixture of less than 0.5 min per plate.

6. Reaction system, wherein the reaction system further comprises at least one reactive absorber and optionally a second reactor for the production of 5-(2-methylmercaptoethyl)-hydantoin.

7. Reaction system according to Claim 5, wherein the reactive absorber is a jet washer system.

8. Reaction system according to one of Claims 1 to 7, wherein zirconium is used in the reactive-rectification column and/or in the reactive absorber and/or in the second reactor as material for parts in contact with the product.

9. Method for continuous production of a methionine salt, wherein the following steps are carried out:
- reaction of 3-methylmercaptopropionaldehyde and hydrogen cyanide or of a component that can be produced therefrom, wherein a solution containing 5-(2-methylmercaptoethyl)-hydantoin is obtained;
- alkaline hydrolysis of the 5-(2-methylmercaptoethyl)-hydantoin obtained to a methionine salt in a reactive-rectification column according to one of Claims 1-8, wherein only the solution containing 5-(2-methylmercaptoethyl)-hydantoin is fed on the topmost plate of the reactive-rectification column and an alkaline circulating solution is fed on a plate located under that, preferably on the 2nd plate from the top.

10. Method according to Claim 9, wherein the alkaline circulating solution contains an alkali carbonate, preferably potassium carbonate.

11. Method according to Claim 9 or 10, wherein water, ammonia and CO₂ are removed from the top of the reactive-rectification column and the NH₃ removed is condensed completely or partially and used in the synthesis of 5-(2-methylmercaptoethyl)-hydantoin.

12. Method according to one of Claims 9 to 11, wherein the concentration of ammonia in the bottom of the rectification column is less than 120 ppm, preferably less than 100 ppm and most preferably less than 80 ppm.

13. Method according to one of Claims 9 to 12, wherein the reaction to 5-(2-methylmercaptoethyl)-hydantoin is carried out in a reactive absorber and then in a second reactor, preferably in a second reactor designed as a flow tube.

14. Method according to one of Claims 9 to 13, wherein the temperature of the reaction mixture at the outlet of the reactive-rectification column is in the range from 180°C to 190°C.

15. Method according to one of Claims 9 to 14, wherein the temperature of the gas phase at the top of the reactive-rectification column is in the range from 160°C to 170°C.

16. Method according to one of Claims 9 to 15, wherein alkaline hydrolysis is carried out at a pressure in the range from 8 bar(excess) to 10 bar(excess).

17. Method according to one of Claims 9 to 16, wherein steam is used as heating and stripping medium in the reactive-rectification column.

18. Method according to one of Claims 9 to 17, wherein the method is carried out in a reaction system according to one of Claims 1 to 8.

19. Use of a reaction system according to one of Claims 1 to 8 for the production of methionine.

## Revendications

1. Système de réaction pour la fabrication d'un sel de méthionine, comprenant une colonne de rectification réactive ayant une hauteur de déversoir dans la plage allant de 100 à 1 000 mm, dans lequel
les écarts entre les plateaux se situent dans la plage allant de 500 à 1 000 mm,
le rapport entre le diamètre de colonne et la longueur de déversoir se situe dans la plage allant de 1,1 à 1, 3,
le rapport entre la surface de section transversale et la surface traversée par le gaz se situe dans la plage allant de 1,5 à 2, et
le nombre de plateaux se situe dans la plage allant de 15 à 25, de préférence dans la plage allant de 18 à 20.

2. Système de réaction selon la revendication 1, dans lequel la hauteur de déversoir se situe dans la plage allant de 150 à 700 mm.

3. Système de réaction selon la revendication 1 ou 2, dans lequel la colonne de rectification réactive est une colonne à plateaux perforés, une colonne à plateaux à fentes, une colonne à plateaux à soupapes ou une colonne à plateaux à calottes.

4. Système de réaction selon la revendication 3, dans lequel le rapport de la somme des surfaces de tous les trous/la surface traversée par le gaz dans la colonne de rectification réactive se situe dans la plage allant de 0,04 à 0,08, et
le diamètre des trous individuels dans le plateau perforé se situe dans la plage allant de 5 à 10 mm.

5. Système de réaction selon l'une quelconque des revendications précédentes, dans lequel les hauteurs de déversoir assurent un temps de séjour moyen du mélange respectif de moins de 0,5 minute par plateau.

6. Système de réaction, le système de réaction comprenant en outre au moins un absorbeur réactif et éventuellement un réacteur secondaire pour la fabrication de 5-(2-méthylmercaptoéthyl)-hydantoine.

7. Système de réaction selon la revendication 5, dans lequel l'absorbeur réactif est un système d'épurateur à jet.

8. Système de réaction selon l'une quelconque des revendications 1 à 7, dans lequel du zirconium est utilisé dans la colonne de rectification réactive et/ou dans l'absorbeur réactif et/ou dans le réacteur secondaire en tant que matériau pour les parties en contact avec le produit.

9. Procédé de fabrication continue d'un sel de méthionine, dans lequel les étapes suivantes sont réalisées :
- la mise en réaction de 3-méthylmercaptopropionaldéhyde et de cyanure d'hydrogène ou d'un composant pouvant être fabriqué à partir de celui-ci, une solution contenant de la 5-(2-méthylmercaptoéthyl)-hydantoine étant obtenue ;
- l'hydrolyse alcaline de la 5-(2-méthylmercaptoéthyl)-hydantoine obtenue en un sel de méthionine dans une colonne de rectification réactive selon l'une quelconque des revendications 1 à 8, seule la solution contenant de la 5-(2-méthylmercaptoéthyl)-hydantoïne étant introduite sur le plateau le plus supérieur de la colonne de rectification réactive, et une solution de circulation alcaline étant introduite sur un plateau situé en dessous, de préférence sur le 2^{e} plateau à partir du haut.

10. Procédé selon la revendication 9, dans lequel la solution de circulation alcaline contient un carbonate alcalin, de préférence du carbonate de potassium.

11. Procédé selon la revendication 9 ou 10, dans lequel de l'eau, de l'ammoniac et du CO₂ sont éliminés par la tête de la colonne de rectification réactive, et le NH₃ éliminé est condensé en totalité ou en partie dans la synthèse de la 5-(2-méthylmercaptoéthyl)-hydantoïne.

12. Procédé selon l'une quelconque des revendications 9 à 11, dans lequel la concentration de l'ammoniac dans le fond de la colonne de rectification est inférieure à 120 ppm, de préférence inférieure à 100 ppm et de manière préférée entre toutes inférieure à 80 ppm.

13. Procédé selon l'une quelconque des revendications 9 à 12, dans lequel la mise en réaction pour former la 5-(2-méthylmercaptoéthyl)-hydantoïne est réalisée dans un absorbeur réactif, puis dans un réacteur secondaire, de préférence dans un réacteur secondaire configuré sous la forme d'un tube d'écoulement.

14. Procédé selon l'une quelconque des revendications 9 à 13, dans lequel la température du mélange réactionnel à la sortie de la colonne de rectification réactive se situe dans la plage allant de 180 °C à 190 °C.

15. Procédé selon l'une quelconque des revendications 9 à 14, dans lequel la température de la phase gazeuse à la tête de la colonne de rectification réactive se situe dans la plage allant de 160 °C à 170 °C.

16. Procédé selon l'une quelconque des revendications 9 à 15 précédentes, dans lequel l'hydrolyse alcaline est réalisée à une pression dans la plage allant de 8 bar (g) à 10 bar (g).

17. Procédé selon l'une quelconque des revendications 9 à 16, dans lequel de la vapeur d'eau est utilisée en tant que milieu de chauffage et d'extraction dans la colonne de rectification réactive.

18. Procédé selon l'une quelconque des revendications 9 à 17, le procédé étant réalisé dans un système de réaction selon l'une quelconque des revendications 1 à 8.

19. Utilisation d'un système de réaction selon l'une quelconque des revendications 1 à 8 pour la fabrication de méthionine.
